# EUROPEAN PATENT APPLICATION

(11) **EP 2 530 176 A1**
(43) Date of publication of application: **05.12.2012**
(21) Application number: 11461518.0
(22) Date of filing: 01.06.2011
(51) Int. Cl.: C22C 14/00, C22F 1/18

(54) **Metal alloy and use thereof**

(71) Applicant: Instytut Fizyki Jadrowej Pan Im. Henryka Niewodniczanskiego, 31-342 Kraków (PL)
(72) Inventor: Jaworski, Jacek Andrzej, 31-342 Krakow (PL); Fleury, Eric, 31-342 Krakow (PL); Kwon, Oh-jip, 31-342 Krakow (PL); Jayamani, Jayaraj, 31-342 Krakow (PL)
(74) Representative: Pawlowski, Adam

(57) **Abstract**

A metallic alloy comprising Ti, Zr, Nb, containing an amorphous phase and a quasicrystalline phase and is represented by the formula:

TiₐZr_{b}Nb_{c}M_{d}Iₑ,

wherein:
- M represents an element selected from a group consisting of Ni, Co, Fe, Mn,
- I represents impurities,
- coefficients a, b, c, d, e represent atomic %, and are equal to: 40 ≤ a ≤ 55, 5 ≤ b ≤ 30, 5 ≤ c ≤ 25 , 5 ≤ d ≤ 30, e ≤ 1.

## Description

The object of the invention is a metal alloy, useful in particular as a biomaterial.

Biomaterials must imperatively obey to specific criteria regarding the physical, chemical and mechanical properties, such as mechanical durability, chemical inertness, resistance to corrosion, bio-adhesion.

Titanium alloys have demonstrated superior biocompatibility among candidate metallic biomaterials however this class of alloys exhibits inferior tribological properties than those of for example Cr-Co-Mo alloys.

The mechanical properties and biocompatibility of titanium alloys can be improved by forming them as metallic glass having amorphous or nanocrystalline structure. Recent progresses in the field of research has demonstrated that amorphous and quasicrystalline phases can be prepared for several compositions based on combinations of titanium, zirconium and metals such as palladium, cobalt, nickel, copper: Ti-Zr-(Ni,Co,Pd), Ti-Zr-(Ni,Pd), Ti-Zr-(Ni,Co) and Ti-Zr-Co. However, these metallic glasses did not comply with all requirements of biocompatibility. Moreover, palladium is an expensive material. An exemplary metallic glass of this type is Ti₄₀Zr₁₀Cu₃₆Pd₁₄ described in publications: Fengxiang Qin, Masahiro Yoshimura, Xinming Wang, Shengli Zhu, Asahi Kawashima, Katsuhiko Asami i Akihisa Inoue "Corrosion Behavior of a Ti-Based Bulk Metallic Glass and Its Crystalline Alloys" (MATERIALS TRANSACTIONS, Vol. 48 (2007), No. 7 pp.1855-1858) and F.X. Qin, X.M. Wang and A. Inoue "Effect of annealing on microstructure and mechanical property of a Ti-Zr-Cu-Pd bulk metallic glass" (Intermetallics, Volume 15, Issue 10, October 2007, Pages 1337-1342). Recent research works concentrate on metallic glasses based on titanium and zirconium, such as Zr₅₅Al₁₀Ni₅Cu₃₀, Zr_{100-*x*-*y*}(Cu_{z}Ag_{1-z})*_{y}*Al*ₓ* (wherein x = 7-9 at.%, y = 42-50 at.% and z = 0,75 - 0,875 at. %), Zr₅₃CO_{18.5}Al_{23.5}Ag₅, Zr₆₀Nb₅Cu₂₀Fe₅Al₁₀ , Zr₆₀Ti₆Cu₁₉Fe₅Al₁₀, Zr₆₀Nb₅Cu_{22.5}Pd₅Al_{7.5}, as well as metallic glasses based on magnesium and zinc, i.e. Mg₆₆Zn₃₀Ca₄ i Mg₇₀Zn₂₅Ca₅ oraz Ti_{43.3}Zr_{21.7}Ni_{7.5}Be_{27.5}, Ti₄₅Zr_{50-*x*}Pd*ₓ*Si₅ (wherein x = 35, 40, 45 at.%). Research results show that only metallic glasses of the type Ti₄₅Zr₃₈Ni₁₇ have a potential of use as biomaterials (H. Lefaix, P. Vermaut, S. Zanna, A. Galtayries, F. Prima, R. Portier, "Structural and Functional (Superficial), Biocompability of New Amorphous/Quasicrystalline Ti-Base Composites", Tenth Annual Conference Yucomat 2008, PL.S.III.2, p. 36, H. Lefaix, A. Asselin, P. Vermaut, J.-M. Sautier, A. Berdal, R. Portier, F. Prima, " On the biocompatibility of a novel Ti-based amorphous composite: structural characterization and in-vitro osteoblasts response", J Mater Sci: Mater. Med. 19 (2008) pp. 1861-1869).

Introducing niobium to metallic alloys based on titanium improved their corrosion resistance in different electrolytes. Ti₆₄Zr₅Fe₆Si₁₇MO₆Nb₂ and Ti₇₀Zr₆Fe₇Si₁₇ metallic glasses are compared by Chunxiang Cui, Ling Bai, Qingzhou Wang, Shaojing Bu and Yumin Qi in "Fabrication of Ti-based amorphous composite and biocompatibility research" (Journal of Wuhan University of Technology--Materials Science Edition, 6 February 2010). The properties of metallic glass (Ti₄₀Zr₁₀Cu₃₆Pd₁₄)_{100-*x*}Nb*ₓ* (*x* = 1, 3, 5 at.%) containing nanoparticles are described by F.X. Qin, X.M. Wang, G.Q. Xie and A. Inoue "Distinct plastic strain of Ni-free Ti-Zr-Cu-Pd-Nb bulk metallic glasses with potential for biomedical applications" (Intermetallics Volume 16, Issue 8, August 2008, Pages 1026-1030).

The enhanced corrosion resistance in different electrolytes and good biocompatibility have also been reported in a few of other systems with the addition of niobium, including Zr₅₉Cu₂₀Al₁₀Ni₈Nb₃, (Cu₆₀Zr₃₀Ti₁₀)₉₅Nb₅, Zr₅₅Al_{20-*x*}CO₂₅Nb*ₓ* (*x* = 0 to 5 at.%) and (Zr₆₀Nb₅)Cu_{17.5}Ni₁₀Al_{7.5}. Also, the addition of niobium can enhanced the stability of the quasicrystalline phase in some Zr-based alloy compositions, i.e., (Zr₆₅Al_{7.5}Cu_{27.5})₉₅Nb₅ and Zr₅₈Al₉Ni₉Cu₁₄Nb₁₀. However, these metallic glasses were still not appropriate for biomedical applications, due to low hardness and low wear resistance (Jeong-Jung Oak, Akihisa Inoue, "Formation, mechanical properties and corrosion resistance of Ti-Pd base glassy alloys", Journal of Non-Crystalline Solids, vol. 354, year 2008, pp. 1828-1832, Yu-Lai Gao, Jun Shen, Jian-Fei Sun, Gang Wang, Da-Wei Xing, Heng-Ze Xian and Bi-De Zhou, "Crystallization behavior of ZrAlNicu bulk metallic glass with wide supercooled liquid region", Materials Letters, vol. 57, year 2003, pp. 1894-1898).

The object of the present invention is to provide a new titanium-based metallic alloy with a good biocompatibility.

A metallic alloy according to the invention comprises Ti, Zr, Nb and contains an amorphous phase and a quasicrystalline phase and is represented by the formula:

TiₐZr_{b}Nb_{c}M_{d}lₑ,

wherein:
- M represents an element selected from a group consisting of Ni, Co, Fe, Mn,
- I represents impurities,
- coefficients a, b, c, d, e represent atomic %, and are equal to: 40 ≤ a ≤ 55,5 ≤ b ≤ 30, 5 ≤ c ≤ 25 , 5 ≤ d ≤ 30, e ≤ 1.

The quasicrystalline phase can be formed by icosahedral crystals, preferably having size ranging from a few nanometers to 100 micrometers.

The quasicrystalline phase can form from 1% to 80% volumetric fraction of the alloy.

The alloy can be formed by a method of the group containing melt spinning, rapid cooling by squeezing, thermal sputtering - cooling from a gaseous phase, ion sputtering techniques.

The object of the invention is also use of the alloy according to the invention for manufacturing a product intended as an implant for human or animal body.

The invention also relates to an implant for implantation in the human or animal body comprising the alloy according to the invention.

The object of the invention is shown by means of exemplary embodiments on a drawing, in which:
Fig. 1 shows the structure of a metallic alloy according to the invention, analyzed by X-ray diffraction (XRD)
Figs. 2-9 show plots of current density for different alloys according to the invention.
Fig. 10 shows a plot of current density for Ti-Zr-Ni alloy.

A metallic alloy according to the invention was obtained on the basis of Ti-Zr-Ni and Ti-Zr-Co alloys, by partial substitution of Zr by Nb and substitution of Ni by Fe and Mn.

The alloy according to the invention contains an amorphous phase and a quasicrystalline phase and is represented by the following formula:

TiₐZr_{b}Nb_{c}M_{d}Iₑ

wherein:
- M represents an element selected from a group consisting of Ni, Co, Fe, Mn,
- I represents impurities,
- coefficients a, b, c, d, e represent atomic %, and are equal to: 40 ≤ a ≤ 55, 5 ≤ b ≤ 30, 5 ≤ c ≤ 25 , 5 ≤ d ≤ 30, e ≤ 1.

The amorphous phase provides good corrosion resistance and the quasictysalline phase provides high hardness of the alloy.

Preparing alloys comprising an amorphous phase and quasicrystalline phase, comprising Ni, Co, Fe, Mn, depends on various factors, such as the electron structure of the elements. For alloys according to the invention, there are maintained appropriate concentrations of metals of groups 4 and 5 (i.e. Ti, Zr, Nb) and groups from 7 to 10 (i.e. Ni, Co, Fe and Mn). The used elements Ni, Co, Fe i Mn are easily accessible and affordable. Optionally, Ni could be replaced by Pd and Pt, Co by Rh and Ir, Fe by Ru and Os, Mn by Tc and Re, but these elements are not common (Tc is not present in the Earth soil) and are expensive. Cu has not been used due to low corrosion resistance.

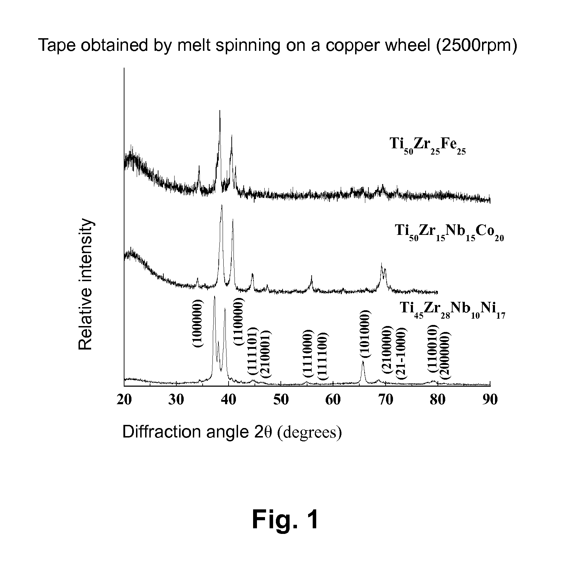

The structure of the layers as analyzed by X-ray diffraction indicated the formation of icosahedral quasicrystalline phase, as shown in Fig. 1. The quasicrystalline phase, i.e. a form of a solid body, in which atoms are aligned in a seemingly regular, but a nonperiodic structure, which makes it impossible to distinguish their elementary cells, has been formed by quasicrystals having sizes from a few nanometers to about 100 micrometers, embedded in an amorphous matrix. The volume fraction of the icosahedral quasicrystalline phase in the metallic glass is dependent on the alloy composition and can be adjusted in order to comply with the desired properties, ranging from 1 to 80%.The volume fraction of the icosahedral quasicrystalline phase can be controlled by the alloy composition and the speed of cooling (which can be adjusted e.g. by chaning the speed of rotation in the melt spinning method).

The alloy according to the invention shows excellent corrosion resistance, and therefore is very suitable for use in medicine as part of implants.

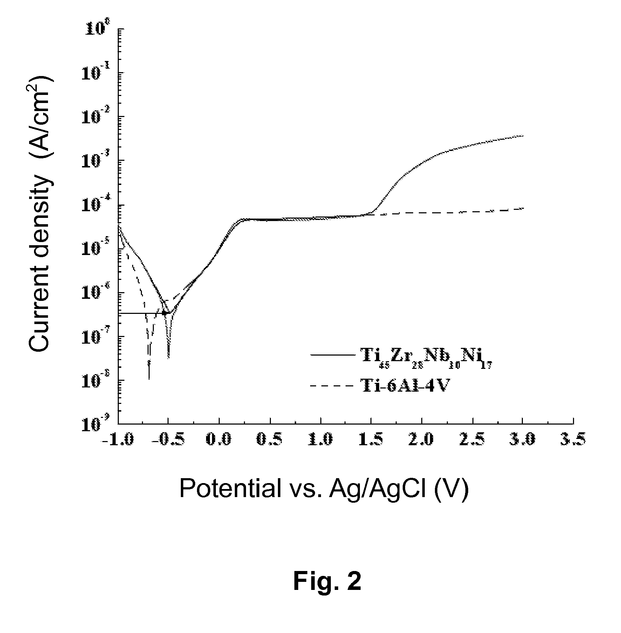

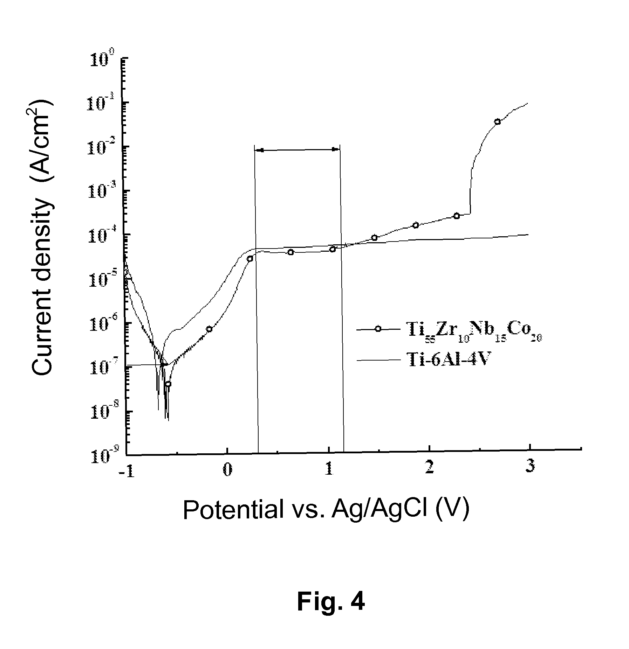

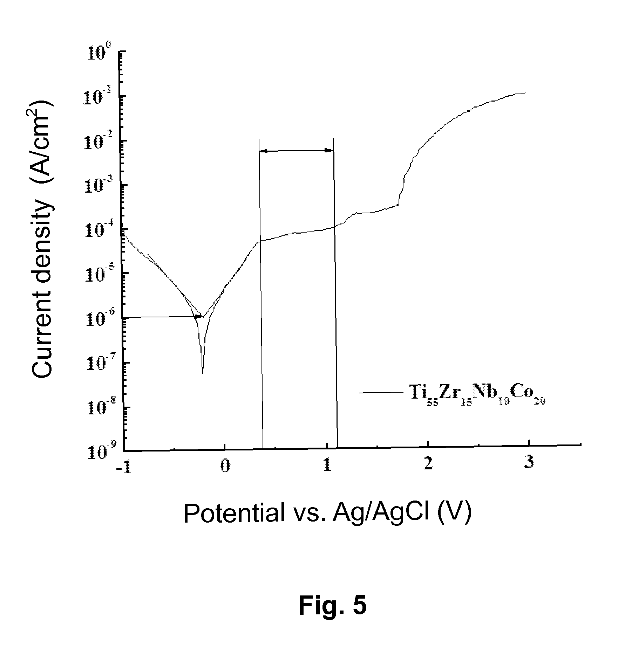

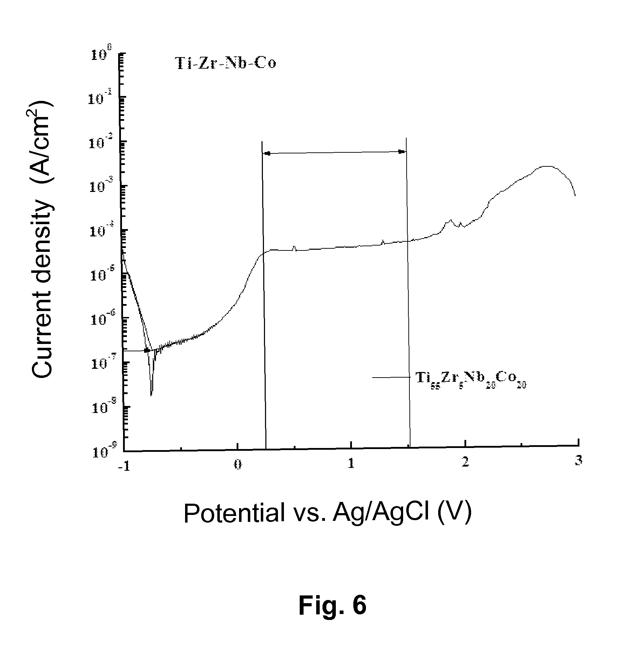

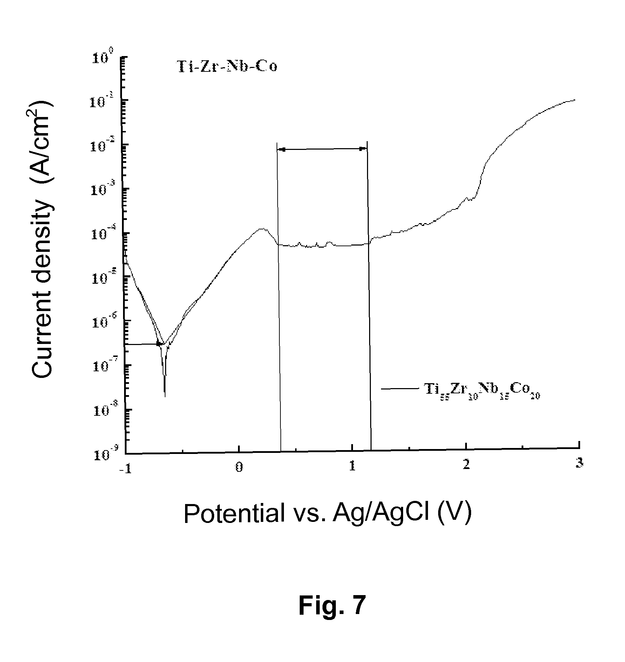

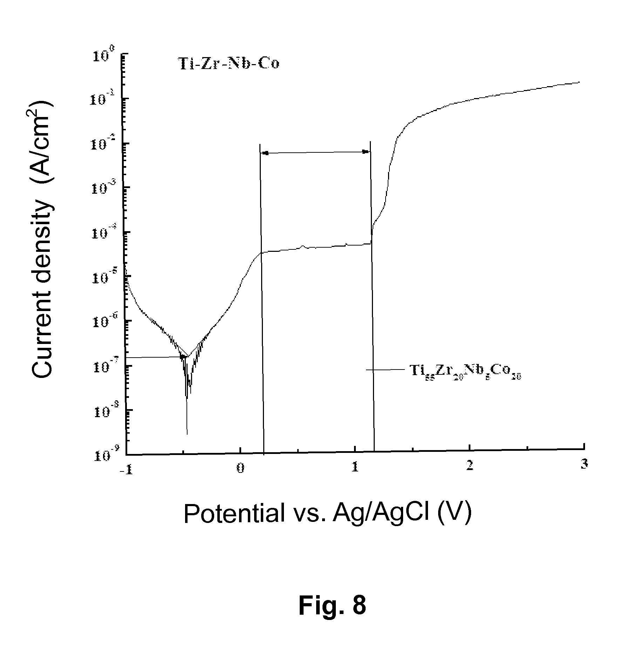

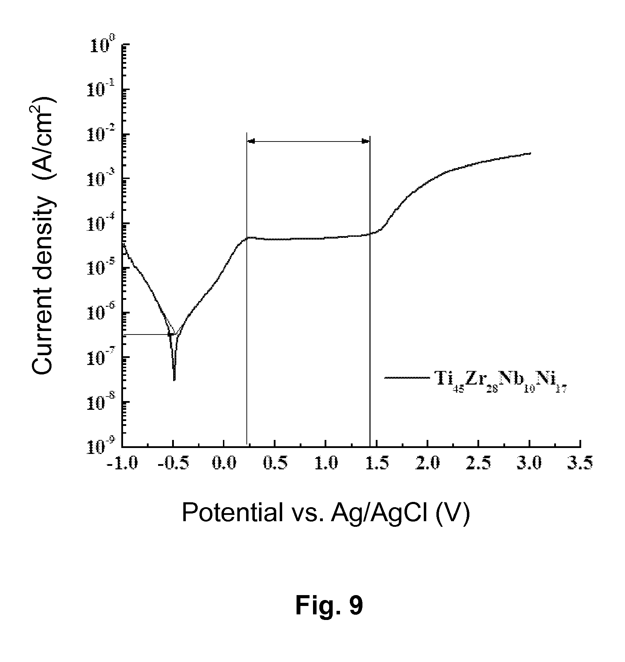

The corrosion properties of the alloy according to the invention have been investigated in simulated physiological solution at 37°C (aerated Hanks' balanced salt solution; 8 NaCl, 0,4 KCl, 0,35 NaHCO₃, 0,25 NaH₂PO₄ x H₂O, 0,06 Na₂HPO₄ x H₂O, 0,19 CaCl₂ x 2 H₂O, 0,19 MgCl₂, 0,06 MgSO₄ x 7H₂O, 1 glukoza, w g/l) by means of potentio-dynamic test. The layers exhibit a low value of the corrosion current density in the range (1- 5)*10⁻⁶ A/cm² i and passivation current den sity In the range (6 - 7)*10⁻⁵ A/cm². The corrosion current density has been measured by a Tofel extrapolation method, wherein exemplary plots for various embodiments of the alloys are presented in Figs. 2-9.

The alloy according to the invention exhibits high wear resistance and increased hardness, as indicated by the measurements below:

| Alloy | Vickers microhardness |
|---|---|
| Ti₄₅Zr₂₈ Nb₁₀Ni₁₇ | 414 |
| Ti₅₀ Zr₁₅Nb₁₅Co₂₀ | 406 |
| Ti₆₅Zr₁₀Nb₁₀Fe₁₅ | 525 |
| Ti₅₀Zr₁₀Nb₁₅Mn₂₅ | 495 |
| Ti₄₅Zr₃₈Ni₁₇ | 396 |
| Ti₆₅Zr₁₀ Fe₂₅ | 375 |
| Ti₅₀Zr₂₅Mn₂₅ | 344 |

As indicated by the measurements in the table above, partial replacement of Fe and Mn by Nb increases the hardness of the alloys by 30% for Fe alloys and by 40% for Mn alloys.

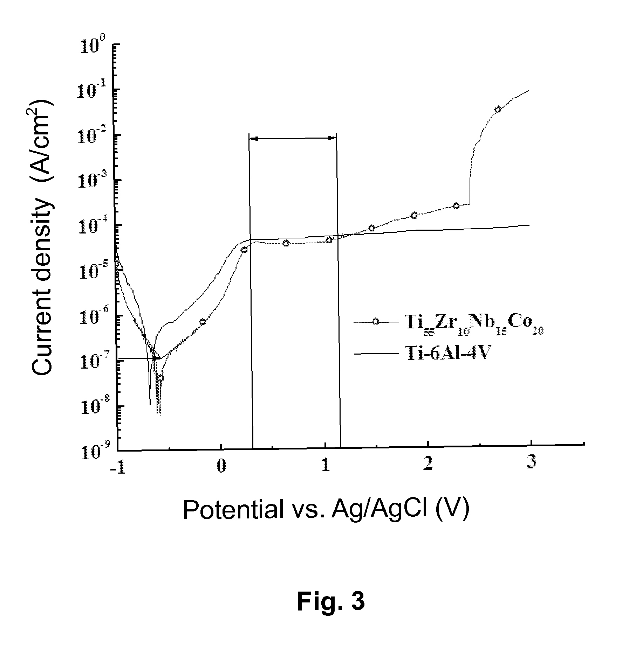

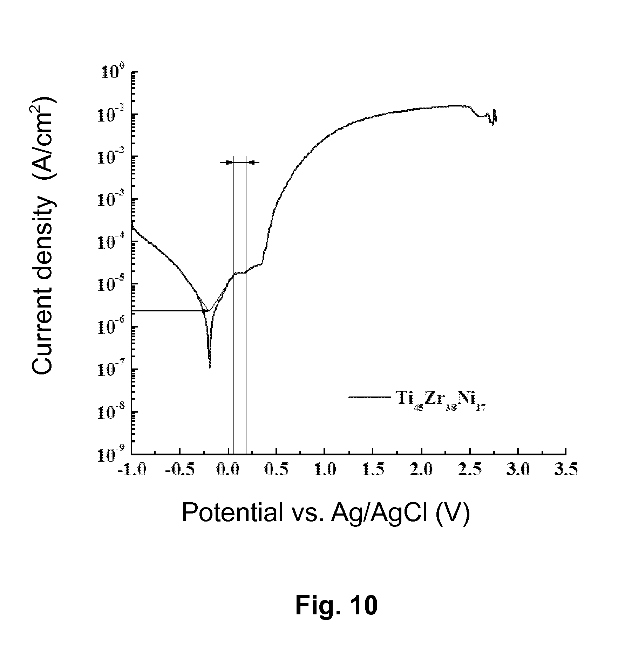

In addition, the alloys according to the invention, as shown in Figs. 3-9, are characterized by a wide range of passivation, i.e. from 1,0 to 1,5V, in contrast to an alloy which does not contain niobium, as shown in Fig. 10, for which the range of passivation equals only 0,2 V. This property is particularly important in relation to corrosion, i.e. then titanium is in contact with other metallic materials.

The partial replacement of Zr by Nb provides a extension of the passivation region to a large value of the potential up to about 1.5V.

The alloy according to the invention can be manufactured by the methods known for metallic glasses, for example by the melt spinning method descrived by Cahn [W. Cahn, Physical Metallurgy, Third edition, Elsevier Science Publishers B.V., 1983] and Liebermann [Liebermann H. and Graham C., Production Of Amorphous Alloy Ribbons And Effects Of Apparatus Parameters On Ribbon Dimensions, IEEE Transactions on Magnetics, Vol Mag-12, No 6, 1976, pp. 921 - 923], by rapid cooling by squeezing as described in the Polish patent application PL384142 or by melt spinning according to the Polish patent application PL378301, as well as by thermal sputtering - cooling from a gaseous phase or by ion sputtering techniques.

## Claims

1. A metallic alloy comprising Ti, Zr, Nb, **characterized In that** it contains an amorphous phase and a quasicrystalline phase and is represented by the formula:
TiₐZr_{b}Nb_{c}M_{d}Iₑ,
wherein:
- M represents an element selected from a group consisting of Ni, Co, Fe, Mn,
- I represents impurities,
- coefficients a, b, c, d, e represent atomic %, and are equal to: 40 ≤ a ≤ 55, 5 ≤ b ≤ 30, 5 ≤ c ≤ 25 , 5 ≤ d ≤ 30, e ≤ 1.

2. The metallic alloy according to claim 1, **characterized in that** the quasicrystalline phase is formed by icosahedral crystals.

3. The metallic alloy according to claim 1, **characterized in that** the quasicrystalline phase is formed by quasicrystals having size ranging from a few nanometers to 100 micrometers.

4. The metallic alloy according to claim 1, **characterized in that** the quasicrystalline phase forms from 1% to 80% volumetric fraction of the alloy.

5. The metallic alloy according to claim 1, **characterized in that** it is formed by a method of the group containing melt spinning, rapid cooling by squeezing, thermal sputtering - cooling from a gaseous phase, ion sputtering techniques.

6. Use of the alloy according to any of claims 1-5 for manufacturing a product intended as an implant for human or animal body.

7. An implant for implantation in the human or animal body comprising an alloy according to any of claims 1-5.
